# EUROPEAN PATENT APPLICATION

(11) **EP 4 316 463 A1**
(43) Date of publication of application: **07.02.2024**
(21) Application number: 22306166.4
(22) Date of filing: 02.08.2022
(51) Int. Cl.: A61K 9/00, A61K 9/16, A61K 9/20, A61K 31/49, A61K 31/51, A61P 31/04, A61P 31/18

(54) **NOVEL FORMULATION**

(71) Applicant: GlaxoSmithKline Intellectual Property Development Limited, Brentford, Middlesex TW8 9GS (GB)
(72) Inventor: TRIDON, Claire, Ware, SG12 0DP (GB); EDWARDS, Andrew, Ware, SG12 0DP (GB); MILLICAN, Sally, Ware, SG12 0DP (GB)
(74) Representative: Plasseraud IP

(57) **Abstract**

An oral formulation of granules of active pharmaceutical ingredient(s) which disperse rapidly in water are presented. The granules generate a palatable oral suspension in solution with good mouthfeel and are particularly useful for the paediatric or hard-of-swallowing patient population.

## Description

### FIELD OF THE INVENTION

The present disclosure relates to a free flowing oral granule formulation of pharmaceuticals capable of dispersing in water rapidly.

### BACKGROUND TO THE INVENTION

Whilst most adult patients are able to swallow conventional tablets and capsules, there remains a portion of patients for whom this presents difficulties, particularly with larger tablets. Paediatric patients, particularly those under 8 years old, frequently experience problems, as do geriatric patients and patients with certain conditions affecting swallowing (for example, neurological patients, patients with a naso-gastric tube and those with particular conditions, such as head and neck cancer).

Alternative formulation types known in the art include suspensions, dispersible granules or powder, dispersible tablets and orodispersible tablets. Dispersible granules, powder or tablets disperse in water (or potentially, other suitable vehicles like milk or juice) to form a suspension which may be drunk by the patient.

Accordingly, granules that can rapidly be dispersed in water and generate a palatable suspension with acceptable mouthfeel are highly desirable.

(2*R*)-2-({4-[(3,4-Dihydro-2*H*-pyrano[2,3-*c*]pyridin-6-ylmethyl)amino]-1-piperidinyl}methyl)-1,2-dihydro-3*H*,8*H*-2a,5,8a-triazaacenaphthylene-3,8-dione (hereinafter "gepotidacin") is an antibiotic in development, which selectively inhibits bacterial DNA gyrase and topoisomerase IV by a unique and novel mechanism. International Patent Application Publication No. WO 2008/128942 describes a series of compounds which can be used as antibacterial agents, including gepotidacin. There is a need to develop alternative formulations for oral drugs such as gepotidacin that can generate a palatable suspension for the paediatric or hard-of-swallowing patient population.

### SUMMARY OF THE INVENTION

In a first aspect, the invention provides an oral formulation of granules comprising
a) up to 60% (w/w) of one or more active pharmaceutical ingredient;
b) 15-75% (w/w) of a soluble filler;
c) 20-60% (w/w) of microcrystalline cellulose;
d) 2-5% (w/w) of a binder; and
e) 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL.

### DETAILED DESCRIPTION OF THE INVENTION

Ideally, dispersible granules or powder presented in bottles, sachets, stickpacks or the like should be free flowing, to ensure content uniformity and to enable full dose measurement and/or recovery from the container. Additionally the granules or powder should disperse rapidly in liquid for patient convenience. Another important consideration, particularly for the paediatric population, is palatability of the medicine. Whilst this is in part dependent upon the taste of the drug in question, it is also in part dependent upon the texture of the suspension formed (i.e. "mouthfeel").

Gepotidacin is prone to consolidation, cohesive, has poor flow properties and a bitter taste, which makes it challenging to formulate. It has a high unit dose requirement; for example the tablets currently used in the Phase 3 gepotidacin clinical trials contain 750mg gepotidacin measured as free base, and the proposed clinical oral dose of gepotidacin is 1500 mg twice a day for treating urinary tract infection (UTI) (NCT04020341 and NCT04187144) and 3000 mg twice a day for treating an infection by *Neisseria gonorrhoeae* (e.g. uncomplicated gonorrhea or urogenital gonorrhea) (NCT04010539).

Such high daily doses would require high drug load oral solid formulations, which poses further challenges, especially for the paediatric or hard-of-swallowing patient population. A formulation that overcomes these issues to generate a palatable oral suspension with good mouthfeel is highly desirable.

### DEFINITIONS

A skilled person would understand that the term "an oral formulation of granules" as used herein refers to any oral pharmaceutical dosage form of a mixture of solids reduced to a finely divided physical state, and encompasses powders and granules.

"Granules are ≤250 microns in diameter" means that the granules have a d50 ≤250 microns. The term d50 has its conventional meaning and can be measured by well-established techniques including sedimentation field flow fractionation, photon correlation spectroscopy, laser diffraction or disk centrifugation. The d50 may be related to volume distribution of the particles and in this case the phrase "the granules are ≤250 microns in diameter" refers to the situation where at least 50% of the volume of the granules have a diameter ≤250 microns. Techniques based on either volume or weight distribution typically result in roughly the same value for the average particle size.

In the context of the invention, the term "soluble filler" refers to a polyol or sugar that exhibits a solubility in water of greater than or equal to 200 mg/ml at 25°C.

In the context of the invention, the term "disintegrant" refers to an excipient which cause the granules to swell and promote disintegration.

In the context of the invention, the term "binder" refers to a water soluble polymer (e.g. with a solubility in water of greater than or equal to 100 mg/mL) that facilitates wet granulation. Typically, the polymer has a molecular weight in the range 500 Da to 2 MDa and an apparent viscosity in the range 3 to 15 mPa•s when in a 2% aqueous solution at 20°C.

The terms "approximate", "approximately" or "about" as used herein in connection with a numerical value are meant to have their usual meaning in the context of the numerical value. Where necessary the words "approximate", "approximately" or "about" may be replaced by the numerical value +/-5%. As used herein, when any of these terms is before a list of numbers, the term applies to each of the listed numbers.

Unless otherwise indicated, the weight percentage is calculated by using the weight of the active ingredient (e.g., gepotidacin free base, gepotidacin mesylate anhydrate or gepotidacin mesylate dihydrate) against the weight of the granules.

The granule Compressibility Index or Carr's Index is measured using a bulk and tapped density test described in USP <1174>. As a skilled person would be aware, Carr's Index is used as an indicator of the compressibility of granules or powder. The term "FFc" refers to the granule flow function and is measured by shear cell technique also described in USP <1174>. Both techniques are used to characterize the flow properties of the granules.

### STATEMENT OF THE INVENTION

The present invention provides an oral formulation of granules comprising
a) up to about 60% (w/w) of one or more active pharmaceutical ingredient;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL.

The granules of the present invention are particularly suitable for active pharmaceutical ingredients typically administered to paediatric patients (particularly those under 6 years old, 8 years old or 12 years old), for geriatric patients and for patients with certain conditions affecting swallowing (for example, neurological patients, patients with a naso-gastric tube and those with particular conditions, such as head and neck cancer).

The granules of the present invention disperse rapidly in water. Dispersion time can be measured using a quantity of granules equivalent to one unit dose of the active pharmaceutical ingredient (e.g. 500mg equivalent gepotidacin free base, or 750 mg equivalent gepotidacin free base) dispersed in 15 mL of water using gentle swirling in a 60 mL dosing cup. As shown in the Examples, the granules of the invention show complete dispersion in water in less than 3 minutes. In one embodiment, about 1100mg to 1800mg of granules of the present invention disperse completely in 15 mL of water using gentle swirling in a 60 mL dosing cup in 3 minutes or less at room temperature. In one embodiment, about 1100mg of granules of the present invention disperse completely in 15 mL of water using gentle swirling in a 60 mL dosing cup in 3 minutes or less at room temperature. In one embodiment, about 1800mg of granules of the present invention disperse completely in 15 mL of water using gentle swirling in a 60 mL dosing cup in 3 minutes or less at room temperature. In one embodiment, about 1000mg of granules of the present invention disperse completely in 15 mL of water using gentle swirling in a 60 mL dosing cup in 3 minutes or less at room temperature. In one embodiment, about 1500mg of granules of the present invention disperse completely in 15 mL of water using gentle swirling in a 60 mL dosing cup in 3 minutes or less at room temperature.

Thus in one embodiment, the present invention provides an oral formulation of granules comprising
a) up to about 60% (w/w) of one or more active pharmaceutical ingredient;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL,
and wherein one unit dose of the granules disperses completely in 15 mL of water using gentle swirling in a 60 mL dosing cup in 3 minutes or less.

As shown in the Examples, the granules of the invention disperse completely in water in less than 3 minutes. In one embodiment, one unit dose of the granules of the invention disperse completely in water in less than 2 minutes. In one embodiment, one unit dose of the granules of the invention disperse completely in water in less than 1 minute. In one embodiment, a dose of the granules comprising 500mg API disperses completely in 15 mL of water using gentle swirling in a 60 mL dosing cup in 3 minutes or less. In one embodiment, a dose of the granules comprising 750mg API disperses in 15 mL of water using gentle swirling in a 60 mL dosing cup in 3 minutes or less.

The granules of the present invention may also be compressed into tablets, in which case dispersion is measured by dispersing 1 to 4 tablets (having a core weight of ≤150 mg) in 5 mL of water, or 5 to 8 tablets are dispersed in 10 mL of water, using gentle swirling for 1-3 min in a 30 mL dosing cup.

The granules of the present invention have good mouthfeel and palatability. Mouthfeel can be measured by standard techniques. For example, the quantity of intra-granular insoluble solids (such as microcrystalline cellulose) may be used as an indication of mouthfeel, or the Pharmacopeia fineness of dispersion test.

The granules are free flowing to ensure weight uniformity and therefore content uniformity and to enable full dose measurement and/or recovery from the container in which they are stored. The free-flowing properties of the granules of the present invention may be characterised by Carr's Index value of ≤30% and an FFc value of ≥10. Thus, in one embodiment the present invention provides an oral formulation of granules comprising
a) up to about 60% (w/w) of one or more active pharmaceutical ingredient;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;
wherein the ratio of microcrystalline cellulose to the binder is ≥5:1; and
wherein the granules are ≤250 microns in diameter with a density of 0.4 - 0.6 g/mL, and have a Carr's Index value of ≤30% and an FFc value of ≥10.

In one embodiment the present invention provides an oral formulation of granules comprising
a) up to about 60% (w/w) of one or more active pharmaceutical ingredient;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL, and have a Carr's Index value of ≤30% and an FFc value of ≥10, and wherein one unit dose of the granules disperses in 15 mL of water using gentle swirling in a 60 mL dosing cup in 3 minutes or less.

The suspension formed by dispersion of the granules of the present invention is liquid and freely flowing.

In order to achieve this desirable combination of properties, the inventors identified a number of critical parameters that are essential for rapid dispersion and palatability. This work is described in the Examples.

The diameter of the granules and the density of the granules are important parameters for rapid dispersion. The diameter of the granules defines the distance that water would need to travel to penetrate the "core" of the granule. Example 1 shows that granules with a median diameter ≤250 microns achieve dispersion within 1 minute. The density of the granules reflects the extent of pores and channels within the granules and also impact dispersion time by providing pores and channels to allow water to rapidly enter the granule. Example 1 shows that a granule density of 0.4 - 0.6 g/mL permits rapid water intake into the granules, leading to granule breakdown and liberation of the active pharmaceutical ingredient. In one embodiment, the granules of the present invention have a granule density of about 0.45 to 0.60 g/mL.

The granule size and density are primarily controlled using the granulation process parameters (e.g. quantity of water for granulation added, the spray rate and the wet massing time) and the oversized granules (e.g > 500 microns) are then further size reduced during the dry milling stage. The granule size and density are also controlled by the grade and level of excipients (the binder and soluble and insoluble fillers).

Example 1 also shows that a granule size of ≤250 microns in diameter disperse quickly in water. In one embodiment, the granules of the present invention have a granule size of about 60 microns to about 250 microns. In one embodiment, the granules of the present invention have a granule size of about 80 microns to about 250 microns.

It is apparent from the above that the physical structure of the granules impacts dispersion speed. The composition of the granules also impacts dispersion speed, as will be discussed further below.

### GRANULE COMPOSITION

The granules of the present invention comprise up to 60% w/w of one or more active pharmaceutical ingredient(s) ("API"). In one embodiment, the granules of the oral formulation of the present invention contain one, two or three different API. In one embodiment, the granules of the oral formulation of the present invention contain one or two different API. In one embodiment, the granules of the oral formulation of the present invention contain two different APIs. In one embodiment, the granules of the oral formulation of the present invention contain only one API.

The skilled reader will readily appreciate that the granules of the invention rapidly disperse. It is therefore apparent that the physical properties of the API and in particular its solubility will have no impact on the dispersion time. The Examples demonstrate that three very different APIs can be formulated successfully, namely daprodustat (N-[(1,3-dicyclohexyl-6-hydroxy-2,4-dioxo-1,2,3,4-tetrahydro-5-pyrimidinyl)carbony]glycine or *N*-[(1,3-Dicyclohexylhexahydro-2,4,6-trioxopyrimidin-5-yl)carbonyl]glycine)), cabotegravir ((3R,6S)-N-[(2,4-difluorophenyl)methyl]-10-hydroxy-6-methyl-8,11-dioxo-4-oxa-1,7-diazatricyclo[7.4.0.03,7]trideca-9,12-diene-12-carboxamide) and gepotidacin. These APIs have a wide range of solubility from mostly insoluble to very soluble (daprodustat 0.074mg/mL, cabotegravir 2.9 mg/mL, gepotidacin 175 mg/mL, all in water at 25°C). The Examples therefore show that diverse APIs can be formulated in the granules of the invention and exhibit rapid dispersion, independent of their solubility in water.

Accordingly, in one embodiment, the granules of the present invention comprise about 0.1 to 60% w/w active pharmaceutical ingredient. In one embodiment, the granules of the present invention comprise about 0.1 to 1% w/w active pharmaceutical ingredient. In one embodiment, the granules of the present invention comprise about 2 to 9% w/w active pharmaceutical ingredient. In one embodiment, the granules of the present invention comprise about 40 to 60% w/w active pharmaceutical ingredient. In one embodiment, the granules of the present invention comprise about 45 to 60% w/w active pharmaceutical ingredient. In one embodiment, the granules of the present invention comprise about 50 to 60% w/w active pharmaceutical ingredient. In one embodiment, the granules of the present invention comprise about 55 to 60% w/w active pharmaceutical ingredient.

In one embodiment, the active pharmaceutical ingredient in the granules of the present invention is gepotidacin or a pharmaceutically acceptable salt thereof. In another embodiment, the active pharmaceutical ingredient in the granules of the present invention is daprodustat or a pharmaceutically acceptable salt thereof. In another embodiment, the active pharmaceutical ingredient in the granules of the present invention is cabotegravir or a pharmaceutically acceptable salt thereof.

The granules of the present invention may be prepared by any suitable method, such as direct compression, dry granulation or wet granulation. In one embodiment, a wet granulation process is employed.

The skilled reader would appreciate that wet granulation could be used to form the granules of the present invention. Compared to other formulation techniques (e.g., direct compression and dry granulation), wet granulation is complicated and usually only performed when other techniques are not suitable. Accordingly, in one embodiment, the API in the oral formulation of the present invention is an API that is not suitable for formulation via direct compression or dry granulation using a low shear blending process.

Typically, wet granulation is particularly suitable in situations where drug loading is low, as this technique ensures that the drug substance is uniformly distributed and locked in the granules. In addition, granules produced by wet granulation are usually better flowing material than ungranulated powders (used in direct compression) or roller compacted granules. This also contributes to content uniformity because better flowing granules ensure better weight control during further processing into tablets, stickpacks, sachets or bottles. Thus the present invention also provides a method of manufacturing granules of the present invention by wet granulation. Granules produced by wet granulation also provide additional taste masking by decreasing the drug surface area in contact with taste buds.

WO2016/12058 discloses a dispersible tablet of bedaquiline fumarate, in which silicified microcrystalline cellulose is used intragranularly. However, the use of silicified microcrystalline cellulose intragranularly is believed to interfere with the process of wet granulation limiting the control of this process that is necessary to achieve granules of the appropriate size and density. Indeed, it is noted that in WO2016/12058, the process of granule formation was complicated involving a separate binder fraction comprised of binder and wetting agent, and an "intragranular" fraction comprised of the active ingredient, silicified microcrystalline cellulose, disintegrant and glidant. When silicified microcrystalline cellulose and glidant is omitted from the granule, wet granulation is much simpler. Thus in one embodiment, the granules of the oral formulation of the present invention do not contain silicified microcrystalline cellulose (SMCC) or glidant.

In Example 2 below, it is shown that the fastest dispersion time was observed with 20-60% w/w intra-granular microcrystalline cellulose, and this was also observed to improve granulation process robustness. Granules having between 20-30% (w/w) microcrystalline cellulose have the best mouthfeel and palatability. Accordingly, in one embodiment, the invention provides an oral formulation of granules comprising between 20-30% (w/w) microcrystalline cellulose. In a more particular embodiment, the invention provides an oral formulation of granules comprising approximately 20% (w/w) microcrystalline cellulose.

A number of different grades of microcrystalline cellulose are available. The skilled person would appreciate that the grade selected should exhibit good compressibility and mouthfeel. Mouthfeel is in part dependent upon the size of the particles. A size of no more than 125 microns is desirable and a size of no more than 50 microns would be ideal. Avicel PH101 and Ceolus KG-1000 are suitable grades. Ceolus KG-1000 is the finest microcrystalline cellulose grade, and results in improved mouthfeel. In one embodiment, the MCC used in the granulate formulation of the present invention is no more than 50 microns in size.

In addition to containing active pharmaceutical ingredient and microcrystalline cellulose, the granules must additionally comprise a binder. The binder may be a natural polymer such as a polysaccharide or polypeptide or a derivative thereof, or a synthetic polymer such as a polyalkylene oxide (e.g. PEG), polyacrylate, polyvinylpyrrolidone, etc. Mixed polymers, e.g. block copolymers and glycopeptides may also be used. In one embodiment, the binder may be selected from the group consisting of hydroxypropylmethylcellulose, povidone, a maltodextrin or starch 1500. In another embodiment, the binder may be selected from the group consisting of hydroxypropylmethylcellulose, and povidone.

In one embodiment, the binder is hydroxypropylmethylcellulose (HPMC), which contains sufficient hydroxypropyl and methoxy groups to render it water-soluble. HPMC having a methoxy degree of substitution from about 19.0 to about 30.0 and a hydroxypropoxy molar substitution from about 4.0 to about 12.0 are generally water-soluble. Methoxy degree of substitution refers to the average number of methyl ether groups present per anhydroglucose unit of the cellulose molecule. Hydroxypropyl molar substitution refers to the average number of moles of propylene oxide which have reacted with each anhydroglucose unit of the cellulose molecule. A preferred HPMC is hypromellose 2910 3 mPa•s or hypromellose 2910 5mPa•s, especially hypromellose 2910 3 mPa•s. Hydroxypropyl methylcellulose is the United States Adopted Name for hypromellose (see Martindale, The Extra Pharmacopoeia, 29th edition, page 1435). In the four digit number "2910", the first two digits represent the approximate percentage of methoxyl groups and the third and fourth digits the approximate percentage composition of hydroxypropoxyl groups; 3 mPa•s or 5 mPa•s is a value indicative of the apparent viscosity of a 2% aqueous solution at 20 °C. In one embodiment, the hydroxypropylmethylcellulose binder is hypromellose 2910 3 mPa•s (i.e. Pharmacoat^{®} 603).

Example 3 shows that the ratio of microcrystalline cellulose: binder should be ≥5:1 in the granule to be fast dispersion. In one embodiment, the invention provides an oral formulation of granules comprising microcrystalline cellulose and a binder selected from HPMC or povidone in a ratio of ≥5:1. In one embodiment, the invention provides an oral formulation of granules comprising microcrystalline cellulose and HPMC in a ratio of ≥5:1. In one embodiment, the invention provides an oral formulation of granules comprising microcrystalline cellulose and binder in a ratio of approximately between 5:1 to 10:1. In one embodiment, the invention provides an oral formulation of granules comprising microcrystalline cellulose and binder in a ratio of approximately 5:1. In one embodiment, the invention provides an oral formulation of granules comprising microcrystalline cellulose and binder in a ratio of approximately 10:1.

The granules additionally contain about 15-75% (w/w) of a soluble filler. In one embodiment, the invention provides an oral formulation of granules comprising microcrystalline cellulose, a binder, one or more soluble fillers and one or more disintegrants, but no additional classes of excipients.

Granules comprising approximately 15 to 75 % w/w soluble filler (e.g. mannitol) have greater mechanical strength (lower level of fines) for downstream processing (e.g. tableting, filling into bottles or sachets/stickpacks). Suitable soluble fillers are polyols, for example, mannitol, sorbitol, maltitol, xylitol, erythritol, isomalt, lactitol and low molecular weight dextrin, and sugars such as lactose (including lactose anhydrous and lactose monohydrate), fructose, sucrose, dextrose and maltose. In one embodiment, the invention provides an oral formulation of granules comprising mannitol. Mannitol is preferred over lactose as it offers better tolerability, especially in the paediatric population. However, it will be appreciated that the active pharmaceutical ingredient may limit the soluble filler selected due to incompatibilities.

The use of soluble fillers in the granule keeps the total levels of insoluble solids in the granules low, to improve mouthfeel. Example 4 shows that 17 to 75 % w/w soluble filler (e.g. mannitol) have greater mechanical strength (lower level of fines) for downstream processing. As will be appreciated, because widely varying levels of the soluble filler are permitted, widely differing levels of active pharmaceutical ingredient can be permitted in the granules, which can be compensated by varying the levels of the soluble filler.

Various grades of mannitol are available. As an example, Pearlitol 160C produces larger and better flowing granules compared to Pearlitol 50C.

The skilled person would appreciate that the total quantity of mannitol in the daily dose (noting that multiple unit doses can be dispersed in vehicle) should be within the recommended daily allowance limit of 50 mg/kg/day (WHO Food additive series Toxicological monograph. 616. Mannitol (WHO Food Additives Series 21) (inchem.org)).

Example 5 shows dispersion of tablets made from granules containing 1.5-15% disintegrant in less than 2 minutes. Any suitable disintegrants may be used in the granule. Suitable disintegrants include cross-linked polyvinylpyrrolidone, modified cellulose gum, e.g. croscarmellose sodium (e.g. Ac-di-Sol), sodium starch glycolate, sodium carboxymethylcellulose, sodium dodecyl sulphate, modified corn starch, microcrystalline cellulose, magnesium aluminium silicate, alginic acid, alginate, powdered cellulose, crospovidone (such as Polyplasdone XL). Other disintegrants that may be considered include Xanthan gum, Gellan gum, soy polysaccharides, and the like. The optimal amount of disintegrant will depend upon which extragranular disintegrant is selected and may be readily determined by those of ordinary skill in the art. Disintegrants cause the granules to swell and promote dispersion.

In one embodiment, the disintegrant is selected from croscarmellose sodium, crospovidone and sodium starch glycolate. In one embodiment, the disintegrant is selected from croscarmellose sodium, crospovidone XL-10 and sodium starch glycolate. In one embodiment, the disintegrant is croscarmellose sodium. In a more particular embodiment, the disintegrant is croscarmellose sodium and it is used in an amount of about 1.5-3% w/w in the granules. In one embodiment, the disintegrant is crospovidone. In a more particular embodiment, the disintegrant is crospovidone and it is used in an amount of about 5-15% w/w in the granule. In one embodiment, the disintegrant is crospovidone XL. In certain embodiments where the disintegrant is crospovidone, a fine grade crospovidone (such as Polyplasdone XL-10) may be used. In one embodiment, the disintegrant is sodium starch glycolate. In a more particular embodiment, the disintegrant is sodium starch glycolate and it is used in an amount of about 3-5% w/w in the granule. It will be appreciated that the active pharmaceutical ingredient may influence the choice of disintegrant based on incompatibilities.

The granules of the present invention may be blended with a sweetener for taste masking and to improve patient acceptability and compliance. Whether a separate sweetener is required is dependent upon factors such as the taste of the active pharmaceutical agent. Taste acceptability is important for once-daily chronic dosing, particularly in the paediatric population. Accordingly, a separate sweetener may be required mixed with the granules of the present invention, but it is noted that some of the possible excipients for use as soluble intragranular fillers are sugars and may also positively impact palatability. Suitable sweeteners include sugars and artificial sweeteners. Five artificial sweeteners are approved by the FDA for children: sucralose, aspartame, neotame, acesulfame potassium and saccharin. In one embodiment, the sweetener is sucralose or neotame. In a more particular embodiment, the sweetener is neotame or sucralose and is used at 1-3% w/w of the oral formulation of the present invention. In the presence of a very bitter drug, sucralose can also be used in combination with acesulfame potassium or neotame or can be replaced by neotame alone which is ~x20 times sweeter than sucralose.

One aspect of the invention is a process for the preparation of the granules of the invention. The granules may be prepared by any suitable method, such as direct compression, dry granulation or wet granulation. In one embodiment, a wet granulation process is employed.

The granules of the present invention may also be processed further to form tablets by compression. The density of the granules of the present invention is not significantly affected by compression to form tablets, and remains within the range of 0.4 - 0.6 g/mL.

The present invention provides an oral formulation of granules comprising
a) up to about 60% (w/w) of one or more active pharmaceutical ingredient;
b) about 15-75% (w/w) of a soluble filler;
c) about 20-60% (w/w) of microcrystalline cellulose;
d) about 2-5% (w/w) of HPMC; and
e) about 1.5-15% (w/w) of a disintegrant;

wherein the ratio of microcrystalline cellulose to HPMC is ≥5:1; and
wherein the granules are ≤250 microns in diameter with a density of 0.4 - 0.6 g/mL.

The present invention provides an oral formulation of granules comprising
a) up to about 60% (w/w) of one or more active pharmaceutical ingredient;
b) about 15-75% (w/w) of mannitol;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL.

In one embodiment, the present invention provides an oral formulation of granules comprising
a) up to about 60% (w/w) of one or more active pharmaceutical ingredient;
b) about 15-75% (w/w) of mannitol;
c) about 20-60% (w/w) of microcrystalline cellulose;
d) about 2-5% (w/w) of HPMC; and
e) about 1.5-15% (w/w) of a disintegrant selected from croscarmellose sodium, sodium starch glycolate or crospovidone;

wherein the ratio of microcrystalline cellulose to HPMC is ≥5:1; and
wherein the granules are ≤250 microns in diameter with a density of 0.4 - 0.6 g/mL.

In one embodiment, the present invention provides an oral formulation of granules comprising
a) about 0.1-60% (w/w) of one or more active pharmaceutical ingredient;
b) about 15-75% (w/w) of mannitol;
c) about 20-60% (w/w) of microcrystalline cellulose;
d) about 2-5% (w/w) of HPMC; and
e) about 1.5-15% (w/w) of a disintegrant selected from croscarmellose sodium, sodium starch glycolate or crospovidone;

wherein the ratio of microcrystalline cellulose to HPMC is ≥5:1; and
wherein the granules are ≤250 microns in diameter with a density of 0.4 - 0.6 g/mL.

In one embodiment, the present invention provides an oral formulation of granules comprising
a) up to about 60% (w/w) of one or more active pharmaceutical ingredient;
b) about 15-75% (w/w) of mannitol;
c) about 20-30% (w/w) of microcrystalline cellulose;
d) about 2-5% (w/w) of HPMC; and
e) about 1.5-15% (w/w) of a disintegrant selected from croscarmellose sodium, sodium starch glycolate or crospovidone;

wherein the ratio of microcrystalline cellulose to HPMC is ≥5:1; and
wherein the granules are ≤250 microns in diameter with a density of 0.4 - 0.6 g/mL.
wherein the granules are ≤250 microns in diameter with a density of 0.4 - 0.6 g/mL.

In one embodiment, the present invention provides an oral formulation of granules comprising
a) about 0.1-60% (w/w) of one or more active pharmaceutical ingredient;
b) about 15-75% (w/w) of mannitol;
c) about 20-30% (w/w) of microcrystalline cellulose;
d) about 2-5% (w/w) of HPMC; and
e) about 1.5-15% (w/w) of a disintegrant selected from croscarmellose sodium, sodium starch glycolate or crospovidone;

wherein the ratio of microcrystalline cellulose to HPMC is ≥5:1; and
wherein the granules are ≤250 microns in diameter with a density of 0.4 - 0.6 g/mL.

In one embodiment, the present invention provides an oral formulation of granules comprising
a) up to about 60% (w/w) of one or more active pharmaceutical ingredient;
b) about 15-75% (w/w) of mannitol;
c) about 20-30% (w/w) of microcrystalline cellulose;
d) about 2-5% (w/w) of HPMC; and
e) about 1.5-15% (w/w) of a disintegrant selected from croscarmellose sodium, sodium starch glycolate or crospovidone;

wherein the ratio of microcrystalline cellulose to HPMC is 5:1 to 10:1; and
wherein the granules are ≤250 microns in diameter with a density of 0.4 - 0.6 g/mL.

In one embodiment, the present invention provides an oral formulation of granules comprising
a) about 0.1-60% (w/w) of one or more active pharmaceutical ingredient;
b) about 15-75% (w/w) of mannitol;
c) about 20-30% (w/w) of microcrystalline cellulose;
d) about 2-5% (w/w) of HPMC; and
e) about 1.5-15% (w/w) of a disintegrant selected from croscarmellose sodium, sodium starch glycolate or crospovidone;

wherein the ratio of microcrystalline cellulose to HPMC is 5:1 to 10:1; and
wherein the granules are ≤250 microns in diameter with a density of 0.4 - 0.6 g/mL.

In one embodiment, any of the above formulations of the present invention additionally comprises about 1-3% (w/w) of a sweetener.

In one embodiment, the granules of the present invention are presented in a single-use stickpack or a sachet. In another embodiment, the granules of the present invention are presented in a single-use or multi-use bottle or container. Thus the present invention provides a container such as a stickpack, a sachet, a bottle or other container containing the granules of the present invention.

### Gepotidacin, cabotegravir and daprodustat granules

In one embodiment, the granules of the present invention comprise gepotidacin or a pharmaceutically acceptable salt thereof.

International Patent Application Publication No. WO 2021/219637 (incorporated herein in its entirety) describes certain crystalline forms of gepotidacin, including gepotidacin mesylate dihydrate, gepotidacin mesylate anhydrate, gepotidacin mesylate monohydrate and gepotidacin anhydrate (free base).

In one embodiment, the granules of the present invention comprise gepotidacin mesylate dihydrate, gepotidacin mesylate anhydrate and/or gepotidacin anhydrate.

In one embodiment, the granules of the present invention comprise gepotidacin mesylate dihydrate.

In one embodiment, the granules of the present invention comprise about 40-60% w/w gepotidacin or a pharmaceutically acceptable salt thereof. In one embodiment, the granules of the present invention comprise about 45-60% w/w gepotidacin or a pharmaceutically acceptable salt thereof. In one embodiment, the granules of the present invention comprise about 50-60% w/w gepotidacin or a pharmaceutically acceptable salt thereof. In one embodiment, the granules of the present invention comprise about 55-60% w/w gepotidacin or a pharmaceutically acceptable salt thereof. In one embodiment, the granules of the present invention comprise about 60% w/w gepotidacin or a pharmaceutically acceptable salt thereof. In one embodiment, the granules of the present invention comprise about 55, 56, 57, 58, 59 or 60% w/w gepotidacin or a pharmaceutically acceptable salt thereof.

In one embodiment, the granules of the present invention comprise about 40-60% w/w gepotidacin mesylate dihydrate. In one embodiment, the granules of the present invention comprise about 45-60% w/w gepotidacin mesylate dihydrate. In one embodiment, the granules of the present invention comprise about 50-60% w/w gepotidacin mesylate dihydrate. In one embodiment, the granules of the present invention comprise about 55-60% w/w gepotidacin mesylate dihydrate. In one embodiment, the granules of the present invention comprise about 55, 56, 57, 58, 59 or 60% w/w gepotidacin mesylate dihydrate. In one embodiment, the granules of the present invention comprise about 60% w/w gepotidacin mesylate dihydrate.

Thus in one embodiment, the present invention provides an oral formulation of granules comprising
a) up to about 60% (w/w) of gepotidacin or a pharmaceutically acceptable salt thereof;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL.

In one embodiment, the granules comprise about 40-60% (w/w) of gepotidacin or a pharmaceutically acceptable salt thereof. In one embodiment, the granules comprise about 45-60% (w/w) of gepotidacin or a pharmaceutically acceptable salt thereof. In one embodiment, the granules comprise about 50-60% (w/w) of gepotidacin or a pharmaceutically acceptable salt thereof. In one embodiment, the granules comprise about 55-60% (w/w) of gepotidacin or a pharmaceutically acceptable salt thereof.

In one embodiment, the soluble filler is mannitol.

In one embodiment, the binder is HPMC.

In one embodiment, the disintegrant is selected from croscarmellose sodium, sodium starch glycolate or crospovidone. In one embodiment, the disintegrant is croscarmellose sodium.

In one embodiment, the ratio of microcrystalline cellulose to HPMC is 5:1 to 10:1.

Thus in one embodiment, the present invention provides an oral formulation of granules comprising
a) up to about 60% (w/w) of gepotidacin or a pharmaceutically acceptable salt thereof;
b) about 15-75% (w/w) of mannitol;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL.

In one embodiment, the present invention provides an oral formulation of granules comprising
a) up to about 60% (w/w) of gepotidacin or a pharmaceutically acceptable salt thereof;
b) about 15-75% (w/w) of a soluble filler;
c) about 20-60% (w/w) of microcrystalline cellulose;
d) about 2-5% (w/w) of HPMC; and
e) about 1.5-15% (w/w) of a disintegrant;

wherein the ratio of microcrystalline cellulose to HPMC is ≥5:1; and
wherein the granules are ≤250 microns in diameter with a density of 0.4 - 0.6 g/mL.

In one embodiment, the present invention provides an oral formulation of granules comprising
a) up to about 60% (w/w) of gepotidacin or a pharmaceutically acceptable salt thereof;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is 5:1 to 10:1; and
wherein the granules are ≤250 microns in diameter with a density of 0.4 - 0.6 g/mL.

In one embodiment, the present invention provides an oral formulation of granules comprising
a) up to about 60% (w/w) of gepotidacin or a pharmaceutically acceptable salt thereof;
b) about 15-75% (w/w) of a soluble filler;
c) about 20-30% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL.

In one embodiment, the present invention provides an oral formulation of granules comprising
a) about 50-60% (w/w) of gepotidacin or a pharmaceutically acceptable salt thereof;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL.

In one embodiment, the present invention provides an oral formulation of gepotidacin granules comprising:
a) about 50-60% (w/w) of gepotidacin or a pharmaceutically acceptable salt thereof;
b) about 15-75% (w/w) of mannitol;
c) about 20-60% (w/w) of microcrystalline cellulose;
d) about 2-5% (w/w) of HPMC; and
e) about 1.5-3% (w/w) of croscarmellose sodium;

wherein the ratio of microcrystalline cellulose to HPMC is ≥5:1; and
wherein the granules are ≤250 microns in diameter with a density of 0.4 - 0.6 g/mL.

In one embodiment, the present invention provides an oral formulation of gepotidacin granules comprising:
a) about 50-60% (w/w) of gepotidacin or a pharmaceutically acceptable salt thereof;
b) about 15-75% (w/w) of mannitol;
c) about 20-60% (w/w) of microcrystalline cellulose;
d) about 2-5% (w/w) of HPMC; and
e) about 1.5-3% (w/w) of croscarmellose sodium;

wherein the ratio of microcrystalline cellulose to HPMC is 5:1 to 10:1; and
wherein the granules are ≤250 microns in diameter with a density of 0.4 - 0.6 g/mL.

In one embodiment, the oral formulation of gepotidacin of the present invention additionally comprise a sweetener. In one embodiment the sweetener is added at about 1-3% (w/w) of the oral formulation. In one embodiment the sweetener is added after the granules of the present invention are formed (i.e. extra-granularly) and blended with the granules. In one embodiment, neotame is added and blended with the gepotidacin granules of the present invention. In one embodiment, neotame is added at about 1% w/w.

In one embodiment, the present invention provides an oral formulation of gepotidacin granules consisting of:
a) about 50-60% (w/w) of gepotidacin or a pharmaceutically acceptable salt thereof;
b) about 15-75% (w/w) of mannitol;
c) about 20-60% (w/w) of microcrystalline cellulose;
d) about 2-5% (w/w) of HPMC; and
e) about 1.5-3% (w/w) of croscarmellose sodium;

wherein the ratio of microcrystalline cellulose to HPMC is ≥5:1; and
wherein the granules are ≤250 microns in diameter with a density of 0.4 - 0.6 g/mL;
and wherein optionally the granules are blended with about 1-3% (w/w) of a sweetener.

In embodiment, the present invention provides an oral formulation of gepotidacin granules wherein a unit dose of the granules comprising 500mg or 750mg of gepotidacin (measured as free base) disperses completely in 15 mL of water using gentle swirling in a 60 mL dosing cup in 3 minutes or less.

In one embodiment, the oral formulation of gepotidacin or a pharmaceutically acceptable salt thereof the present invention is presented in a single-use stickpack or a sachet. Thus the present invention provides a single-use stickpack or a sachet containing the granules of the present invention comprising gepotidacin or a pharmaceutically acceptable salt thereof. In one embodiment, the stickpack or sachet contains a dose of 200mg, 225mg, 250mg, 275mg, 300mg, 325mg, 350mg, 375mg, 400mg, 425mg, 450mg, 475mg, 500mg, 525mg, 550mg, 575mg, 600mg, 625mg, 650mg, 675mg, 700mg, 725mg, 750mg, 775mg or 800mg of gepotidacin (measured as free base). In one embodiment, the stickpack or sachet contains a unit dose of 325, 500, 650 or 750 mg of gepotidacin (measured as free base). In one embodiment, the stickpack or sachet contains a unit dose of 500 or 750 mg of gepotidacin (measured as free base).

The present invention also provides a bottle or a container containing the granules of the present invention comprising gepotidacin or a pharmaceutically acceptable salt thereof. Thus in another embodiment, the oral formulation of gepotidacin or a pharmaceutically acceptable salt thereof is in a single-use bottle or container containing 1500 mg of gepotidacin (measured as free base).

In one embodiment, the granules of the present invention comprise daprodustat or a pharmaceutically acceptable salt thereof. Daprodustat is the USAN, INN and JAN name for the compound N-[(1,3-dicyclohexyl-6-hydroxy-2,4-dioxo-1,2,3,4-tetrahydro-5-pyrimidinyl)carbony]glycine (the IUPAC name for this compound is *N*-[(1,3-Dicyclohexylhexahydro-2,4,6-trioxopyrimidin-5-yl)carbonyl]glycine). Daprodustat is a HIF prolyl hydroxylase inhibitor that is in development for the treatment of anemia associated with chronic kidney disease in patients on dialysis and not on dialysis. Anemia associated with chronic kidney disease does occur rarely in children, including babies as young as 3 months. As mentioned above, dialysis patients need to restrict volume intake, so for daprodustat, a dispersible oral formulation (e.g. granules or tablets) capable of being rapidly dispersed in very small volumes that generate a palatable suspension are highly desirable. Thus in one embodiment, the present invention provides an oral formulation of granules comprising daprodustat or a pharmaceutically acceptable salt thereof, wherein a unit dose of the granules disperse in 5 mL of water using gentle swirling for 1-3 min in a 30 mL dosing cup. The unit dose of granules may be in the form of tablets.

Daprodustat exhibits keto/enol tautomerism. All tautomers of daprodustat, including mixtures thereof, are intended to be encompassed within the scope of the invention.

Thus in one embodiment, the present invention provides an oral formulation of granules comprising
a) up to about 60% (w/w) of daprodustat or a pharmaceutically acceptable salt thereof;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL.

In one embodiment, the granules of the present invention comprise cabotegravir or a pharmaceutically acceptable salt thereof, such as cabotegravir sodium. Thus in one embodiment, the present invention provides an oral formulation of granules comprising
a) up to about 60% (w/w) of cabotegravir or a pharmaceutically acceptable salt thereof;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;

wherein the ratio of microcrystalline cellulose to the binder is ≥5:1; and
wherein the granules are ≤250 microns in diameter with a density of 0.4 - 0.6 g/mL.

In one embodiment, the present invention provides the following oral formulations of granules:

| Component | Daprodustat (% w/w) | | | Gepotidacin (% w/w | Cabotegravir sodium (% w/w) |
|---|---|---|---|---|---|
| Strength (mg) | 0.25^{∗} | 1.5^{∗} | 2^{∗} | 500 or 750^{∗∗} unit dose | 5 |
| API | 0.3 | 2.0 | 2.7 | 57.8 | 7.9 |
| Mannitol (soluble filler) | 74.7 | 73.0 | 72.3 | 15.2 | 55.1 |
| Microcrystalline Cellulose (soluble filler) | 20.0 | 20.0 | 20.0 | 20.0 | 30.0 |
| Disintegrant | 3.0^{a} | 3.0^{a} | 3.0^{a} | 3.0^{a} | 4.0^{b} |
| HPMC (binder) | 2.0 | 2.0 | 2.0 | 4.0 | 3.0 |

| | | | | | |
|---|---|---|---|---|---|
| ^{∗}measured as free acid **measured as free base ^{a} croscarmellose sodium ^{b} sodium starch glycolate | | | | | |

### MEDICAL USE

The present invention provides the oral formulation of the present invention for use in therapy.

The present application provides a method for treating a bacterial infection comprising administering to a human subject in need thereof an oral formulation of the present invention comprising gepotidacin or a pharmaceutically acceptable salt thereof. The bacterial infection can be caused by a wide range of organisms including both Gram-negative and Gram-positive organisms, and the infections include, but are not limited to, upper and/or lower respiratory tract infections, skin and soft tissue infections, urinary tract infections, and gonorrhea. In some embodiments, the infection is urinary tract infection. In some embodiments, the infection is gonorrhoea. The method of treating bacterial infections by using gepotidacin is disclosed in WO2008/128942, WO2016/027249 and WO2020/201833, all of which are incorporated herein by reference in their entirety.

In some embodiments, the infection is urinary tract infection caused by *Escherichia coli (E. coli), Staphylococcus saprophyticus, Citrobacter koseri,* or *Klebsiella pneumoniae (K. pneumoniae).* In some embodiments, the infection is urinary tract infection caused by *E. coli.* In another embodiment, the infection is gonorrhoea caused by *Neisseria gonorrhoeae.*

Thus the present invention provides an oral formulation of the present invention comprising gepotidacin or a pharmaceutically acceptable salt thereof for use in the treatment of a bacterial infection in a human subject. In one embodiment the infection is uUTI or gonorrhea. The present invention also provides use of gepotidacin or a pharmaceutically acceptable salt thereof in the manufacture of the oral formulation of the present invention for use in the treatment of a bacterial infection in a human subject. In one embodiment the infection is uUTI or gonorrhea. In another embodiment, the invention provides a method for the treatment of a bacterial infection in a human subject in need thereof, comprising administering to said human the granules of the present invention comprising gepotidacin or a pharmaceutically acceptable salt thereof.

In particular embodiments, the human subject is a paediatric patient, under 18 years of age. In one embodiment, the human subject is a paediatric patient under 12 years of age. In one embodiment, the human subject is a paediatric patient under 6 years of age. In one embodiment, the human subject is a paediatric patient between 6 and 12 years of age. In one embodiment, the human subject is a paediatric patient aged between 3 months and 12 years. In another embodiment, the human subject is a paediatric patient under 8 years of age. In a more particular embodiment, the human subject is a paediatric patient aged between 3 months and 8 years.

In one embodiment, the human subject is a paediatric, adolescent or adult female.

As used herein, the term "treatment" refers to alleviating the specified condition, eliminating or reducing one or more symptoms of the condition, slowing or eliminating the progression of the condition, and preventing or delaying the reoccurrence of the condition in a previously afflicted or diagnosed patient or subject.

As used herein, the term "effective amount" means that amount of a drug or pharmaceutical agent that will elicit the biological or medical response of a tissue, system, animal, or human subject that is being sought, for instance, by a researcher or clinician.

In one embodiment, the present application provides a method for treating urinary tract infection (UTI), comprising administering the granules of the present invention, in a therapeutically effective amount in a human subject in need thereof, wherein the gepotidacin is administered at 500, 750 or 1500 mg twice a day, 6-12 hours apart depending on age group and/or body weight.

In one embodiment, the present application provides a method for treating urinary tract infection (UTI), comprising administering the gepotidacin granules of the present invention, in a therapeutically effective amount in a human subject in need thereof, wherein the subject weighs between 10 to 20 kg and the gepotidacin granules are administered at 500 mg twice a day, 6-12 hours apart for 2, 3, 4 or 5 days. In one embodiment, the present application provides a method for treating urinary tract infection (UTI), comprising administering the gepotidacin granules of the present invention, in a therapeutically effective amount in a human subject in need thereof, wherein the subject weighs between 10 to 20 kg and the gepotidacin granules are administered at 500 mg twice a day, 6-12 hours apart for 5 days.

In one embodiment, the present application provides a method for treating urinary tract infection (UTI), comprising administering the gepotidacin granules of the present invention, in a therapeutically effective amount in a human subject in need thereof, wherein the subject weighs between 20 to 40 kg and the gepotidacin granules are administered at 750 mg twice a day, 6-12 hours apart for 2, 3, 4 or 5 days. In one embodiment, the present application provides a method for treating urinary tract infection (UTI), comprising administering the gepotidacin granules of the present invention, in a therapeutically effective amount in a human subject in need thereof, wherein the subject weighs between 20 to 40 kg and the gepotidacin granules are administered at 750 mg twice a day, 6-12 hours apart for 5 days. In one embodiment, the present application provides a method for treating urinary tract infection (UTI), comprising administering the gepotidacin granules of the present invention, in a therapeutically effective amount in a human subject in need thereof, wherein the subject weighs 40 kg or over and the gepotidacin granules are administered at 1500 mg twice a day, 6-12 hours apart for 2, 3, 4 or 5 days. In one embodiment, the present application provides a method for treating urinary tract infection (UTI), comprising administering the gepotidacin granules of the present invention, in a therapeutically effective amount in a human subject in need thereof, wherein the subject weighs 40 kg or over and the gepotidacin granules are administered at 1500 mg twice a day, 6-12 hours apart for 5 days.

In one embodiment, the present application provides a method for treating urinary tract infection (UTI), comprising administering the gepotidacin granules of the present invention, in a therapeutically effective amount in a human subject in need thereof, wherein the subject is between 2-6 years of age and the gepotidacin granules are administered at 500 mg twice a day, 6-12 hours apart for 2, 3, 4 or 5 days. In one embodiment, the present application provides a method for treating urinary tract infection (UTI), comprising administering the gepotidacin granules of the present invention, in a therapeutically effective amount in a human subject in need thereof, wherein the subject is between 2-6 years of age and the gepotidacin granules are administered at 500 mg twice a day, 6-12 hours apart for 5 days.

In one embodiment, the present application provides a method for treating urinary tract infection (UTI), comprising administering the gepotidacin granules of the present invention, in a therapeutically effective amount in a human subject in need thereof, wherein the subject is between 6-12 years of age and the gepotidacin granules are administered at 750 mg twice a day, 6-12 hours apart for 2, 3, 4 or 5 days. In one embodiment, the present application provides a method for treating urinary tract infection (UTI), comprising administering the gepotidacin granules of the present invention, in a therapeutically effective amount in a human subject in need thereof, wherein the subject is between 6-12 years of age and the gepotidacin granules are administered at 750 mg twice a day, 6-12 hours apart for 5 days.

In one embodiment, the present application provides a method for treating urinary tract infection (UTI), comprising administering the gepotidacin granules of the present invention, in a therapeutically effective amount in a human subject in need thereof, wherein the subject is over 12 years of age and the gepotidacin granules are administered at 1500 mg twice a day, 6-12 hours apart for 2, 3, 4 or 5 days. In one embodiment, the present application provides a method for treating urinary tract infection (UTI), comprising administering the gepotidacin granules of the present invention, in a therapeutically effective amount in a human subject in need thereof, wherein the subject is over 12 years of age and the gepotidacin granules are administered at 500 mg twice a day, 6-12 hours apart for 5 days.

In one embodiment, in any of the above methods of treatment of UTI of the present invention, the twice-a-day dosages are taken approximately 12 hours apart. In one embodiment, the UTI is uncomplicated UTI. In particular, the granules of the present application are presented as a unit dose and taken preferably from 1 to 5 times daily, such as once or twice daily to achieve the desired effect.

In one embodiment, the gepotidacin granules of the present invention is administered twice a day. In one embodiment, gepotidacin is administered for any of 2, 3, 4, 5, 6 or 7 continuous days. In one embodiment, in any aspect of the present application, gepotidacin is administered for 5 continuous days.

In another embodiment, the present application provides a method for treating an infection by *Neisseria gonorrhoeae,* comprising administering the gepotidacin granules disclosed herein in a therapeutically effective amount in a human subject in need thereof, wherein the gepotidacin granules are administered twice, each at 3000 mg, 6-12 hours apart. In one embodiment, the gepotidacin granules are administered twice, each at 3000 mg, 10-12 hours apart.

The granules of daprodustat may be used in therapy, more particularly in the treatment of anemia. In a particular embodiment, the daprodustat granules of the present invention may be used in the treatment of anemia associated with chronic kidney disease (also known as renal anemia). In yet another embodiment, the invention provides use of daprodustat or a pharmaceutically acceptable salt thereof in the manufacture of the daprodustat granules of the present invention for use in the treatment of anemia due to chronic kidney disease. In another embodiment, the invention provides a method for the treatment of anemia due to chronic kidney disease in a subject in need thereof, comprising administering to said subject the daprodustat granules of the invention.

### MANUFACTURE

One aspect of the invention is a process for the preparation of the granules of the invention. The granules may be prepared by any suitable method, such as direct compression, dry granulation or wet granulation. In one embodiment, a wet granulation process is employed. In wet granulation, the granules may be prepared by contacting or mixing the relevant ingredients with a vehicle which may be aqueous or non-aqueous, or a combination. In one embodiment, the vehicle is water (qs), more particularly purified water (qs). Such wet granulation process is in one embodiment, a batch high shear granulation process. Use of a batch high shear wet granulation process results in better control of granule size and a reduced levels of fines compared to top spray granulation or dry granulation (i.e. roller compaction).

As shown in Example 4, the use of a soluble filler in the granule improves wet granulation and granules comprising 15 to 75 % w/w soluble filler (e.g. mannitol) have greater mechanical strength (lower level of fines) for downstream processing.

Use of a batch high shear wet granulation process results in improved granule properties (flow properties, control of particle size distribution, level of fines) and improved granules palatability by reducing the drug surface area in contact with the taste buds.

### EXAMPLES

### Protocols for measurement of dispersion time:

- Method 1 (granules): Dispersion time is measured using the patient instruction for use method where the quantity of granules equivalent to one unit dose of the API (e.g. 750 mg equivalent gepotidacin free base) is dispersed in 15 mL of water using gentle swirling in a 60 mL dosing cup. The time to form a uniform fine dispersion is measured.
- Method 2 (tablets): disintegration is measured using USP method <701>
- Method 3 (tablets): dispersion is measured using the patient instruction for use method where 1 to 4 tablets are dispersed in 5 mL of water and where 5 to 8 tablets are dispersed in 10 mL of water using gentle swirling for 1-3 min in a 30 mL dosing cup.

It was observed that dispersion times measured using methods 2 and 3 show good correlation. All examples using granules used Method 1. All examples using tablets used Methods 2 or 3.

### Example 1 - Granule size

The granules are ≤250 microns in diameter with a density of 0.4 - 0.6 g/mL which passes the European Pharmacopeia fineness of dispersion test and which disperses very quickly in water in less than 60s to form a fine uniform dispersion. Granules with a median size of 300 microns are too large and fail European Pharmacopeia fineness of dispersion test.

All the granules below have good flow properties and good content uniformity, with a Carr's index of <30% and a FFc value of ≥10.

**Table 1**

| Formulation | Granule median size (microns) | Granule density (g/mL) | Dispersion Time (sec) |
|---|---|---|---|
| 4 | 82 | 0.48 | 73 |
| 4 | 102 | 0.49 | 12 |
| 4 | 120 | 0.47 | 13 |
| 4 | 133 | 0.53 | 56 |
| 4 | 149 | 0.52 | 23 |
| 4 | 163 | 0.60 | 16 |
| 4 | 176 | 0.57 | 12 |
| 4 | 243 | 0.58 | 12 |

The composition of Formulation 4 is shown in Table 2 below.

**Table 2**

| Component | Formulation 4 (% w/w) |
|---|---|
| API (Gepotidacin mesylate dihydrate) | 57.8 |
| Mannitol | 17.2 |
| MCC | 20.0 |
| Hypromellose | 2.0 |
| Croscarmellose sodium | 3.0 |
| Total | 100.0 |

### Example 2 - MCC content

Table 3a shows that granules containing 20 - 60 % w/w microcrystalline cellulose (MCC) disperse in water rapidly. Dispersion test methods 1 was used. The fastest dispersion time for gepotidacin granules was observed with 20% w/w microcrystalline cellulose and this was also optimal for granulation process robustness.

**Table 3a**

| Formulation | API | Quantity of intra-granular Microcrystalline Cellulose (% w/w) | Dosage Form | Dispersion Time (seconds) |
|---|---|---|---|---|
| 4 | Gepotidacin | 20 | Granule | 23 |
| 5 | Gepotidacin | 0 | Granule | 111 |
| 6 | Gepotidacin | 10 | Granule | 118 |

Table 3b shows the rapid dispersion time of tablets made from granules containing 20 - 60 % w/w microcrystalline cellulose (MCC). Dispersion test methods 2 and 3 were used. Although the dosage forms tested were tablets, the granules in the tablets contribute to the rapid dispersion of the tablets because of the size, density and composition of the granules. Compression into tablets can make dispersion of the granules more challenging, but the results still show complete dispersion of the tablets in less than 2 minutes.

**Table 3b**

| Formulation | API | Quantity of intra-granular Microcrystalline Cellulose (% w/w) | Dosage Form | Dispersion Time (seconds) | |
|---|---|---|---|---|---|
| 1 | Daprodustat | 20 | Tablet | | 21 |
| 2 | Daprodustat | 20 | Tablet | | 7 |
| 3 | Cabotegravir | 30 | Tablet | | 30 or 21 |
| 8 | Daprodustat | 60 | Tablet | | 107 |
| 9 | Daprodustat | 60 | Tablet | | 63 |

Table 4 gives the compositions of formulations 1, 2, 3, 4, 5, 6, 8, 9. The intragranular components shown in the table are the components of the granules. The additional tablet components for formulations 1, 2, 3, 8 and 9 are shown as "tablet formulation" in the table.

**Table 4**

| Componen t | Formula tion 1 (% w/w) | Formula tion 2 (% w/w) | Formula tion 3 (% w/w) | Formula tion 4 (% w/w) | Formula tion 5 (% w/w) | Formula tion 6 (% w/w) | Formula tion 8 (% w/w) | Formula tion 9 (% w/w) |
|---|---|---|---|---|---|---|---|---|
| ***Intra-granular formulati on*** | | | | | | | | |
| Drug substance | 0.3 | 2.7 | 7.9 | 57.8 | 57.8 | 57.8 | 0.3 | 3.3 |
| Mannitol | 74.7 | 72.3 | 55.1 | 17.2 | 37.2 | 27.2 | 32.7 | 29.7 |
| MCC | 20.0 | 20.0 | 30.0 | 20.0 | 0 | 10.0 | 60.0 | 60.0 |
| Hypromell ose | 2.0 | 2.0 | 3.0 | 2.0 | 2.0 | 2.0 | 4.0 | 4.0 |
| Croscarme Ilose sodium | 3.0 | 3.0 | 0 | 3.0 | 3.0 | 3.0 | 3.0 | 3.0 |
| Sodium starch glycolate | 0 | 0 | 4.0 | 0 | 0 | 0 | 0 | 0 |
| Total | 100.0 | 100.0 | 100.0 | 100.0 | 100.0 | 100.0 | 100.0 | 100.0 |

| ***Tablet formulati on*** | | | | | | | | |
|---|---|---|---|---|---|---|---|---|
| Granule | 75.0 | 75.0 | 44.4 | - | - | - | 75.1 | 75.1 |
| SMCC/MC C | 20.5 | 20.5 | 42.6 | - | - | - | 20.9 | 21.4 |
| Croscarme Ilose sodium | 3.0 | 3.0 | 0 | - | - | - | 3.0 | 3.0 |
| Crospovid one XL-10 | 0 | 0 | 10.0 | - | - | - | 0 | 0 |
| Sucralose | 1.0 | 1.0 | 1.0 | - | - | - | 0 | 0 |
| Magnesiu m Stearate | 0.5 | 0.5 | 0 | - | - | - | 1.0 | 0.5 |
| Sodium stearyl fumarate | 0 | 0 | 2.0 | - | - | - | 0 | 0 |
| Total | 100.0 | 100.0 | 100.0 | - | - | - | 100.0 | 100.0 |

### Example 3 - MCC binder ratio

Table 5 shows that the gepotidacin granules having an MCC:binder ratio of 5:1 to 10:1 disperse very quickly in water.

**Table 5**

| Formulation | API | MCC: Binder Ratio | Dosage form | Dispersion Time (seconds) |
|---|---|---|---|---|
| 4 | Gepotidacin | 10 : 1 | Granule | 23 |
| 7 | Gepotidacin | 5 : 1 | Granule | < 60 |

The composition of formulation 4 and 7 are in Table 6.

**Table 6**

| Component | Formulation 4 (% w/w) | Formulation 7 (% w/w) |
|---|---|---|
| Drug substance | 57.8 | 57.8 |
| Mannitol | 17.2 | 15.2 |
| MCC | 20.0 | 20.0 |
| Binder (Hypromellose) | 2.0 | 4.0 |
| Croscarmellose sodium | 3.0 | 3.0 |
| Total | 100.0 | 100.0 |

### Example 4 - Soluble filler

Table 7 shows that granules, and tablets made from granules, comprising a second soluble filler in addition to MCC have lower amounts of insoluble solids and therefore an improved mouthfeel.

Formulation 4 was tested both as granules and as tablets. The dispersion time of formulation 4 was measured from granules (without addition of extra-granular components and prior to compression). The dispersion time of the tablet formulations 1, 3, 4 and 8 was measured from tablets dosage form at a target mean tablet tensile strength of 2 MPa (±0.5 MPa). The granules in the tablets contribute to the rapid dispersion of the tablets even when compressed into tablets because of the size, density and composition of the granules. Compression into tablets can make dispersion of the granules more challenging, but the results still show complete dispersion in less than 2 minutes.

**Table 7**

| Formula tion | API | Quantity of soluble intra-granular filler (mannit ol) (% w/w) | Quantity of insoluble intra-granular filler (MCC) (% w/w) | Quantity of intra-granular insoluble solids/MCC (mg/unit dose) - Surrogate for mouthfeel | % fines in the granules - Surrogate for granule mechanical strength, manufacturi ng process robustness | Dosage form | Dispersion Time (seconds) |
|---|---|---|---|---|---|---|---|
| 1 | Da prod u stat | 75 | 20 | 15 | 7.9 | Tablet | 21 |
| 3 | Caboteg ravir | 55 | 30 | 20 | 6.3 | Tablet | 30 |
| 4 | Gepotida cin | 17 | 20 | 145.6 | 2.3 | Tablet | 116 |
| 4 | Gepotida cin | 17 | 20 | 145.6 | 0.45 | Granule | 23 |
| 8 | Da prod u stat | 33 | 60 | 45 | 7.2 | Tablet | 107 |

The composition of formulations 1, 3, 4 and 8 are shown in Table 8. As noted above, formulation 4 was tested both as granules comprising only the intra-granular components below, and separately as a compressed tablet form comprising the tablet formulation components below.

**Table 8**

| Component | Formulation 1 (% w/w) | Formulation 3 (% w/w) | Formulation 4 (% w/w) | Formulation 8 (% w/w) |
|---|---|---|---|---|
| ***Intra-granular formulation*** | | | | |
| Drug substance | 0.3 | 7.9 | 57.8 | 0.3 |
| Mannitol | 74.7 | 55.1 | 17.2 | 32.7 |
| MCC | 20.0 | 30.0 | 20.0 | 60.0 |
| Hypromellose | 2.0 | 3.0 | 2.0 | 4.0 |
| Crosca rmellose sodium | 3.0 | 0 | 3.0 | 3.0 |
| Sodium starch glycolate | 0 | 4.0 | 0 | 0 |
| Total | 100.0 | 100.0 | 100.0 | 100.0 |

| ***Tablet formulation*** | | | | |
|---|---|---|---|---|
| Granule | 75.0 | 44.4 | 56.0 | 75.1 |
| SMCC and/or MCC | 20.5 | 42.6 | 33.0 | 20.9 |
| Crosca rmellose sodium | 3.0 | 0 | 6.0 | 3.0 |
| Crospovidone XL-10 | 0 | 10.0 | 0 | 0 |
| Sucralose | 1.0 | 1.0 | 1.5 | 0 |
| Acesulfame potassium | 0 | 0 | 3.0 | 0 |
| Magnesium Stearate | 0.5 | 0 | 0 | 1.0 |
| Sodium stearyl fumarate | 0 | 2.0 | 0.5 | 0 |
| Total | 100.0 | 100.0 | 100.0 | 100.0 |

### Example 5 - Disintegrant

Table 9 shows that granules comprising an intra-granular disintegrant (e.g. croscarmellose sodium, sodium starch glycolate and crospovidone XL-10) disperse in less than 3 min when compressed into tablets at a target mean tensile strength of 2 MPa (±0.5 MPa). 1.5 - 3% w/w croscarmellose sodium, 5 - 15% w/w crospovidone XL and 3-5% w/w sodium starch glycolate were successfully used in the granules to produce fast dispersing drug products (dispersing in 30s). Test Methods 2 and 3 above were used to measure tablet dispersion/disintegration. Table 9 shows the disintegration time of tablets comprising granules containing optimal levels of disintegrants: 3% w/w croscarmellose sodium, 4% w/w sodium starch glycolate and 5% w/w crospovidone XL-10.

**Table 9**

| Formulation | API | Intra-granular disintegrant | Quantity of intra-granular disintegrant (% w/w) | Tablets Disintegration Time (seconds) |
|---|---|---|---|---|
| 1 | Daprodustat | Crosca rmellose sodium (Ac-Di-Sol^{®}) | 3.0 | 21 |
| 3 | Ca boteg ravi r | Sodium starch glycolate (Glycolys^{®}) | 4.0 | 30 |
| 10 | Daprodustat | Crospovidone XL-10 (Polyplasdone^{®} XL-10) | 5.0 | 105 |

Table 10 shows the compositions of formulations 1, 3 and 13.

**Table 10**

| Component | Formulation 1 (% w/w) | Formulation 3 (% w/w) | Formulation 10 (% w/w) |
|---|---|---|---|
| ***Intra-granular formulation*** | | | |
| Drug substance | 0.3 | 7.9 | 0.1 |
| Mannitol | 74.7 | 55.1 | 31.9 |
| MCC | 20.0 | 30.0 | 60.0 |
| Hypromellose | 2.0 | 3.0 | 0 |
| Povidone | 0 | 0 | 3.0 |
| Croscarmellose sodium | 3.0 | 0 | 0 |
| Sodium starch glycolate | 0 | 4.0 | 0 |
| Crospovidone XL-10 | 0 | 0 | 5.0 |
| Total | 100.0 | 100.0 | 100.0 |

| ***Tablet formulation*** | | | |
|---|---|---|---|
| Granule | 75.0 | 44.4 | 83.3 |
| SMCC and/or MCC | 20.5 | 42.6 | 4.7 |
| Croscarmellose sodium | 3.0 | 0 | 0 |
| Crospovidone XL-10 | 0 | 10.0 | 10.0 |
| Sucralose | 1.0 | 1.0 | 0 |
| Magnesium Stearate | 0.5 | 0 | 0 |
| Sodium stearyl fumarate | 0 | 2.0 | 2.0 |
| Total | 100.0 | 100.0 | 100.0 |

## Claims

1. An oral formulation of granules comprising
a) up to about 60% (w/w) of one or more active pharmaceutical ingredient;
b) about 15-75% (w/w) of a soluble filler;
c) about 20-60% (w/w) of microcrystalline cellulose;
d) about 2-5% (w/w) of a binder; and
e) about 1.5-15% (w/w) of a disintegrant;
wherein the ratio of microcrystalline cellulose to the binder is ≥5:1; and
wherein the granules are ≤250 microns in diameter with a density of 0.4 - 0.6 g/mL.

2. An oral formulation as claimed in claim 1, wherein the granules have a Carr's Index value of ≤30% and an FFc value of ≥10.

3. An oral formulation as claimed in claim 1 or claim 2, wherein one unit dose of the granules disperses completely in 15 mL of water using gentle swirling in a 60 mL dosing cup in 3 minutes or less.

4. An oral formulation as claimed in any of claims 1-3, wherein the binder is HPMC or povidone.

5. An oral formulation as claimed in any of claims 1-4, wherein microcrystalline cellulose and the binder are in a ratio of approximately 5:1 to 10:1.

6. An oral formulation as claimed in any of claims 1-5, wherein the soluble filler is mannitol.

7. An oral formulation as claimed in any of claims 1-6, wherein the disintegrant is selected from croscarmellose sodium, crospovidone and sodium starch glycolate.

8. An oral formulation as claimed in any of claims 1-7, wherein the active pharmaceutical ingredient is gepotidacin or a pharmaceutically acceptable salt thereof.

9. An oral formulation as claimed in 8, wherein the granules comprise gepotidacin mesylate dihydrate.

10. An oral formulation as claimed in any of claims 1-9 for use in therapy.

11. An oral formulation as claimed in claims 8-9 for use in the treatment of a bacterial infection in a human subject.

12. Use of gepotidacin or a pharmaceutically acceptable salt thereof in the manufacture of an oral formulation as claimed in any of claims 1-9 for the treatment of a bacterial infection in a human subject.

13. A method of treating a bacterial infection, comprising administering an oral formulation as claimed in claims 8-9 in a therapeutically effective amount in a human subject in need thereof.

14. A use or method as claimed in claims 12-13, wherein the human subject is a paediatric patient under 12 years of age.

15. A method of manufacturing an oral formulation as claimed in any of claims 1-9, wherein the granules are formed by wet granulation.
